(19) Europäisches Patentamt
European Patent Office
Office européen des brevets

(11) EP 2 070 904 A1

(12) EUROPEAN PATENT APPLICATION

(43) Date of publication:
**17.06.2009 Bulletin 2009/25**

(21) Application number: **07380349.6**

(22) Date of filing: **12.12.2007**

(51) Int Cl.:
***C07C 41/03*** *(2006.01)* ***C07C 209/18*** *(2006.01)*
***C07D 209/12*** *(2006.01)* ***C07F 15/00*** *(2006.01)*

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Laboratorios Del. Dr. Esteve, S.A.**
**08041 Barcelona (ES)**

(72) Inventors:
- **Heller, Detleff**
  **18057 Rostock (DE)**
- **Drexler, Hans-Joachim**
  **18198 Gross-Schwass (DE)**
- **Preetz, Angelika**
  **18057 Rostock (DE)**
- **Torrens Jover, Antoni**
  **08221 Terrasa (Barcelona) (ES)**
- **Buschmann, Helmut Heinrich**
  **08960 San Just Desvern (Barcelona) (ES)**

(74) Representative: **Bernardo Noriega, Francisco**
**ABG Patentes, S.L.**
**Avenida de Burgos, 16D**
**Edificio Euromor**
**28036 Madrid (ES)**

# (54) Rhodium-phosphorus complexes and their use in ring opening reactions

(57) The present invention is directed to novel rhodium-phosphorus complexes of formula:

$[Rh(PP')(solv)_2]X$

the process for their preparation and their use as catalysts in the ring opening reaction of heteronorbomenes and other $\alpha,\beta$-unsaturated compounds.

EP 2 070 904 A1

Printed by Jouve, 75001 PARIS (FR)

## Description

### FIELD OF THE INVENTION

The present invention is directed to rhodium-phosphorus complexes and their use as catalysts in the ring opening reaction of heteronorbomenes and other α,β-unsaturated compounds.

### BACKGROUNG OF THE INVENTION

The efficient construction of stereochemically complex carbocyclic compounds through the ring opening of heterobicyclic alkenes has become an important reaction for C-C and C-X bond formation. Pioneering work in this field as well as the exploration of its synthetic potential in enantioselective synthesis and synthesis of natural products was first described by Lautens and co-workers [For natural product synthesis, see: Lautens, M.; Rovis, T. J. Org. Chem. 1997, 62, 5246-5247. Lautens, M.; Rovis, T. Tetrahedron 1999, 8967-8976. Lautens, M.; Colucci, J. T.; Hiebert, S.; Smith, N. D.; Bouchain, G. Org. Lett. 2002, 4, 1879-1882. Lautens, M.; Fagnou, K.; Zunic, V. Org. Lett. 2002, 4, 3465-3468].

Particular attention has been placed on the desymmetrization of oxobenzonorbomadiene **1**, as the products are precursors to the medicinally important tetrahydronaphthalene moiety [Snyder, S. E.; Aviles-Garay, F. A.; Chakraborti, R.; Nichols, D. E.; Watts, V. J.; Mailman, R. B. J. Med. Chem. 1995, 38, 2395- 2409. Kamal, A.; Gayatri, N. L. Tetrahedron Lett. 1996, 37, 3359- 3362. Kim, K.; Guo, Y.; Sulikowski, G. A. J. Org. Chem. 1995, 60, 6866. Perrone, R.; Berardi, F.; Colabufo, N. A.; Leopoldo, M.; Tortorella, V.; Fiorentini, F.; Olgiati, V.; Ghiglieri, A.; Govoni, S. J. Med. Chem. 1995, 3, 8, 942-949].

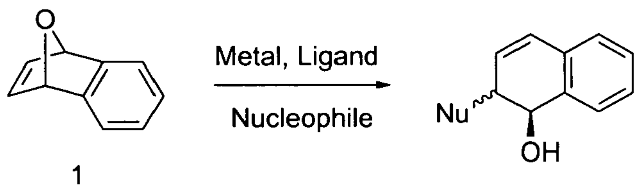


The following scheme shows the huge synthetic potential of oxabenzonorbomadiene **1.**

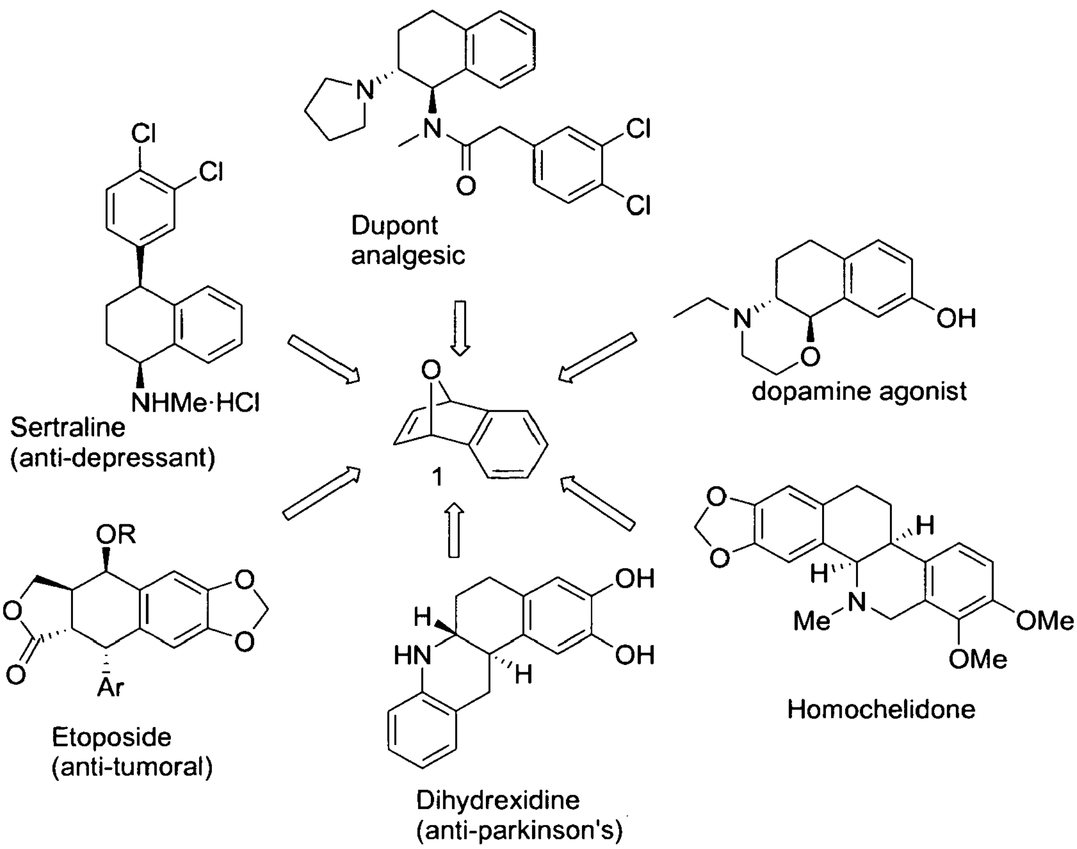

Dupont analgesic

Sertraline (anti-depressant)

dopamine agonist

1

Etoposide (anti-tumoral)

Homochelidone

Dihydrexidine (anti-parkinson's)

Among the carbon nucleophiles capable of inducing ring opening of heterobicyclic alkenes, organolithium [Caple, R.; Chen, G. M.-S.; Nelson, J. D. J. Org. Chem. 1971, 36, 2874-2876. Arjona, O.; de la Pradilla, R. F.; Garcia, E.; Martin-Domenech, A.; Plumet, J. Tetrahedron Lett. 1989, 30, 6437-6440. Lautens, M.; Gajda, C.; Chiu, P. J. Chem. Soc., Chem. Commun. 1993, 1193-1194] and cuprate [Lautens, M.; Smith, A. C.; Abd-El-Aziz, A. S.; Huboux, A. H. Tetrahedron Lett. 1990, 31, 3523] reagents were the first class of nucleophiles used, affording the corresponding *syn* addition products. Later, softer organometallic species such as phenylstannane [Fugami, K.; Hagiwara, S.; Oda, H.; Kosugi, M. Synlett 1998, 477-478], alkylaluminums [Millward, D. B.; Sammis, G.; Waymouth, R. M. J. Org. Chem. 2000, 65, 3902-3909], dialkylzincs [Lautens, M.; Hiebert, S.; Renaud, J.-L. Org. Lett. 2000, 2, 1971-1973. Lautens, M.; Renaud, J.-L.; Hiebert, S. J. Am. Chem. Soc. 2000,122, 1804-1805. Lautens, M.; Hiebert, S.; Renaud, J.-L. J. Am. Chem. Soc. 2001, 123, 6834-6839] alkylzinc halides [Rayabarapu, D. K.; Chiou, C.-F.; Cheng, C.-H. Org. Lett. 2002, 4, 1679-1682] and arylboronic acids [Murakami, M.; Igawa, H. Chem. Commun. 2002, 390-391. Lautens, M.; Dockendorff, C.; Fagnou, K.; Malicki, A. Org. Lett. 2002, 4, 1311-1314] in the presence of a variety of metal catalysts, also proved to be efficient reagents for the *syn*-stereoselective ring-opening addition.

On the other hand, the rhodium-catalyzed asymmetric ring-opening of oxabenzonorbomadiene with alcohols and phenols produces hydronaphtalenes in high yields and with excellent enantioselectivities by means of an *anti* addition [Lautens, M.; Fagnou, K.; Rovis, T. J. Am. Chem. Soc. 2000, 122, 5650. Lautens, M.; Fagnou, K.; Taylor, M. Org. Lett. 2000, 2, 1677. Lautens, M.; Fagnou, K.; Taylor, M.; Rovis, T. J. Organomet. Chem. 2001, 624, 259. Lautens, M.; Fagnou, K.; Hiebert, S. Acc. Chem. Res. 2003, 36, 48]. Also, rhodium-catalyzed ring-openings of oxabicyclic alkenes with amines [Lautens, M.; Fagnou, K. J. Am. Chem. Soc. 2001, 123, 7170], carboxilates [Lautens, M.; Fagnou, K. Tetrahedron 2001, 57, 5067], 1,3-dicarbonyl nucleophiles [Lautens, M.; Fagnou, K.; Yang, D. J. Am. Chem. Soc. 2003, 125, 14884] and sulfur nucleophiles [Leong, P.; Lautens, M. J. Org. Chem. 2004, 69, 2194] have been reported as *anti*-stereoselective reactions.

Azabicyclic alkenes, including azabenzonorbomadienes, were found to be less reactive than the corresponding oxabicyclic alkenes. The first example of the transition metal-catalyzed ring-opening reaction of azabicyclic alkenes is the palladium-catalyzed alkylative ring-opening of *N*-substituted azabenzonorbornadienes [Lautens, M.; Hiebert, S.; Renaud, J. Org. Lett. 2000, 2, 1971. Cabrera, S.; Arrayas, R. G.; Carretero, J. C. Angew. Chem., Int. Ed. 2004, 43, 3944]. Rhodium-catalyzed ring-opening addition of aliphatic and cyclic amines to azabicyclic substrates has also been reported [Lautens, M.; Fagnou, K.; Zunic, V. Org. Lett. 2002, 4, 3465. Cho, Y-h.; Zunic, V.; Senboku, H.; Olsen, M.; Lautens, M. J. Am. Chem. Soc. 2006, 128, 6837].

WO2001030734 (Fagnou, K.; Lautens, M.) discloses a procedure for making an enantiomerically enriched compound containing a hydronaphthalene ring structure. The process involves reacting oxabenzonorbomadiene compounds with nucleophiles using rhodium as a catalyst and in the presence of a phosphine ligand. The compounds synthesized may be used in pharmaceutical preparations. The catalyst used in this document is $[Rh(COD)Cl]_2/PPF\text{-}^tBU_2$.

Nevertheless, Lautens disclosed later a halide exchange protocol in order to achieve better activity and enantioselectivity, specially for other than alcohols or phenolic nucleophiles [Lautens, M.; Fagnou, K.; Yang, D. J. Am. Chem. Soc. 2003, 125, 14884]. Even though the new catalyst, $[RhI(PPF\text{-}^tBu_2)]$, improved the efficiency of such reactions, high temperatures (always 80°C or above) were still required.

On the other hand, EP 1 225 166 (Degussa AG) is directed to enantiomerically enriched *N*-acylated β-aminoacids synthesized by catalytic enantioselective hydrogenation of *E*-isomers and *Z*-isomers of 3-amino acrylic acid derivatives in the presence of a pre-catalyst such as $[Rh(MeDuPHOS)COD]BF_4$. The inventors propose this pre-catalyst is first converted to a solvent complex ($[Rh(MeDuPHOS)(MeOH)_2]BF_4$) which is actually the catalytically active species by prehydrogenation of the diolefinic ligand.

Heller and co-workers have also explored the asymmetric hydrogenation of prochiral substrates in presence of Rh(diolefin) complexes with chiral phosphines. These complexes are hydrogenated in parallel to the asymmetric reaction obtaining thus the true catalytic species, $[Rh(chiral\ diphosphine)(MeOH)_2]BF_4$ [Tetrahedron Lett. 2001, 42, 223; J. Organomet. Chem. 2001, 621, 89; Dalton Trans. 2003, 1606].

It would be highly desirable to develop new catalysts which overcome the problems raised in ring opening reactions. In particular, lower reaction temperatures together with lower amounts of substrates would facilitate the industrial application of these processes.

## BRIEF DESCRIPTION OF THE INVENTION

The authors of the present invention have surprisingly found that a cationic solvent complex, represented by the general formula $[RhPP(solv)_2]X$, presents excellent behaviour in ring opening reactions, improving significantly the results obtained when compared to the complexes previously described in the prior art. In particular, the application of these cationic solvent complexes in the asymmetric version of this reaction (asymmetric ring opening, ARO) provides higher enantioselectivities and complete conversions whereas it allows lowering the substrate/nucleophile ratio. In addition, such complexes enable lower reaction temperatures and shorter reaction times.

A first aspect of the present invention refers to the use of a rhodium-phosphorus complex of formula (I):

$$[Rh(PP)(solv)_2]X \qquad (I)$$

wherein:

- PP is a bidentate phosphorus ligand or two monodentate phosphorus ligands;
- solv is a coordinating solvent; and
- X is an anionic counterion,

as catalyst in a ring opening reaction.

A second aspect of the present invention is a process for the catalytic ring opening of α,β-unsaturated compounds of formula (II) and (III):

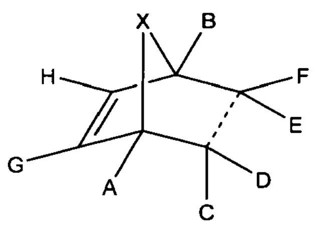
(II)

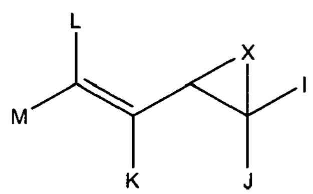
(III)

or a stereoisomer, salt or solvate thereof,

wherein the dotted line represents no bond, a single bond or a double bond;

X is oxygen, sulfur or NR, being R hydrogen, substituted or unsubstituted alkyl, substituted or unsubstituted alkenyl,

substituted or unsubstituted aryl or a suitable amino protecting group;
A, B, D, F, G, H, J, K and L are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine;
C and E are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine; or when the dotted line represents a single bond, they can be bound together forming a 5-7 member aliphatic or aromatic ring, optionally substituted; wherein in case C and E form an aromatic ring, D and F do not exist;
J and M are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine; or they can be bound together forming the compound:

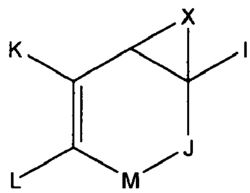

wherein, in this case, J and M are independently selected from substituted or unsubstituted methylene, oxygen, sulfur or NR, being R hydrogen, substituted or unsubstituted alkyl, substituted or unsubstituted alkenyl, substituted or unsubstituted aryl or a suitable amino protecting group; or one of J or M does not exist,
in the presence of a rhodium-phosphorus complex of formula (I) as defined above.

In another aspect the present invention is directed to a rhodium-phosphorus complex of formula (I'):

$$[Rh(PP')(solv)_2]X \qquad (I')$$

wherein

PP' is a metallocene-type diphosphine ligand,
solv is a coordinating solvent, and
X is an anionic counterion.

with the proviso that $[Rh(PPF\text{-}PCy_2)(MeOH)_2]BF_4$ is not included.

Another aspect of the present invention is a process for the preparation of a rhodium-phosphorus complex (I') as defined in the paragraph above, which comprises the hydrogenation of a metal diolefin complex of formula (IV) in the presence of a suitable coordinating solvent (solv),

$$[Rh(PP')(diolefin)]X \qquad (IV)$$

wherein PP', X and solv have the same meanings as defined for (I') and diolefin represents a diolefin molecule or two monoolefin molecules.

According to a further aspect, the present invention refers to the process described in the paragraph above which further comprises the subsequent addition of a compound of formula (II) or (III) as defined previously and a nucleophile to promote the ring opening reaction of said compound of formula (II) or (III).

Finally, another aspect of the present invention is the rhodium-phosphorus complex (I') obtainable by the process as defined above.

## DESCRIPTION OF THE DRAWING

Figure 1 shows the $^{31}P$ NMR spectrum of $[Rh(PPF\text{-}P^tBu_2)(THF)_2]BF_4$.

## DETAILED DESCRIPTION OF THE INVENTION

In the context of the present invention, the following terms have the meaning detailed below:

"Alkyl" refers to a straight or branched hydrocarbon chain radical consisting of carbon and hydrogen atoms, containing no unsaturation, having one to eight carbon atoms, and which is attached to the rest of the molecule by a single bond, e. g., methyl, ethyl, n-propyl, i-propyl, n-butyl, t-butyl or n-pentyl. Alkyl radicals may be optionally substituted by one or more substituents such as an aryl, halo, hydroxy, alkoxy, carboxy, cyano, carbonyl, acyl, alkoxycarbonyl, amino, nitro, mercapto or alkylthio.

"Alkenyl" refers to a straight or branched hydrocarbon chain radical consisting of carbon and hydrogen atoms, containing one or more unsaturated bonds, having at least two carbon atoms and which is attached to the rest of the molecule by a single bond, e. g., vinyl or allyl. Alkenyl radicals may be optionally substituted by one or more substituents such as an aryl, halo, hydroxy, alkoxy, carboxy, cyano, carbonyl, acyl, alkoxycarbonyl, amino, nitro, mercapto or alkylthio.

"Cycloalkyl" refers to a stable 3-to 10-membered monocyclic or bicyclic radical which is saturated or partially saturated, and which consist solely of carbon and hydrogen atoms, such as cyclohexyl or adamantyl. Unless otherwise stated specifically in the specification, the term "cycloalkyl" is meant to include cycloalkyl radicals which are optionally substituted by one or more substituents such as alkyl, halo, hydroxy, amino, cyano, nitro, alkoxy, carboxy or alkoxycarbonyl.

"Aryl" refers to single and multiple aromatic hydrocarbon radicals, including multiple ring radicals that contain separate and/or fused aryl groups. Typical aryl groups contain from 1 to 3 separated or fused rings and from 6 to about 18 carbon ring atoms, such as phenyl, naphthyl, indenyl, fenanthryl or anthracyl radical. The aryl radical may be optionally substituted by one or more substituents such as hydroxy, mercapto, halo, alkyl, phenyl, alkoxy, haloalkyl, nitro, cyano, dialkylamino, aminoalkyl, acyl or alkoxycarbonyl.

"Heterocyclyl" refers to a stable 3- to 15- membered ring which consists of carbon atoms and from one to five heteroatoms selected from the group consisting of nitrogen, oxygen, and sulfur, preferably a 4-to 8-membered ring with one or more heteroatoms, more preferably a 5-or 6-membered ring with one or more heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quatemized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole and tetrahydrofurane.

"Alkoxy" refers to a radical of the formula -ORa where Ra is an alkyl radical as defined above, e. g., methoxy, ethoxy or propoxy. "Aryloxy" refers to a radical of formula -ORb wherein Rb is an aryl radical as defined above.

"Alkylamine" refers to a radical of the formula -NHRa or -NRaRb, optionally quatemized, wherein Ra and Rb are independently an alkyl radical as defined above. The alkyl radical may be optionally substituted by one or more substituents such as an aryl, halo, hydroxy, alkoxy, carboxy, cyano, carbonyl, acyl, alkoxycarbonyl, amino, nitro, mercapto or alkylthio.

"Arylamine" refers to a radical of the formula -NHRa or -NRaRb, optionally quatemized, wherein Ra and Rb are independently an aryl radical as defined above. The aryl radical may be optionally substituted by one or more substituents such as hydroxy, mercapto, halo, alkyl, phenyl, alkoxy, haloalkyl, nitro, cyano, dialkylamino, aminoalkyl, acyl or alkoxycarbonyl.

"Amino protecting group" refers to a group that blocks the $NH_2$ function for further reactions and can be removed under controlled conditions. The amino protecting groups are well known in the art, representative protecting groups are carbamates and amides such as substituted or unsubstituted or substituted acetates. Also different alkyl moeties may serve as amino protecting groups. Additional examples of amino protecting groups can be found in reference books such as Greene and Wuts "Protective Groups in Organic Synthesis", John Wiley & Sons, Inc., New York, 1999.

"Halogen" or "halo" refers to bromo, chloro, iodo or fluoro.

The term "complex" means a molecular structure in which neutral molecules or anions (called ligands) bond to a central metal atom (or ion) by coordinate covalent bonds. Extensive descriptions of terms related to coordination chemistry in reference books such as Robert H. Crabtree "The Organometallic Chemistry of the Transition Metals", Wiley-Interscience; 4 ed., 2005.

The term "catalyst" is recognized in the art and means a substance that increases the rate of a reaction without modifying the overall standard Gibbs energy change in the reaction and without itself being consumed in the reaction. The changing of the reaction rate by use of a catalyst is called catalysis. As used herein, the catalyst is used in a substoichiometric amount relative to a reactant, i. e. a catalytic amount. A preferred catalytic amount is considered herein from 0.0001 to 10 mol% of catalyst relative to the substrate to be opened, more preferably from 0.001 to 1 mol%, more

preferably from 0.005 to 0.05 mol% and even more preferably is 0.01 mol%.

The term "ligand" refers to a molecule or ion that is bonded directly (i.e. covalently) to a metal center.

As used herein in reference to a ligand or metal complex, the term "asymmetric" means that the ligand or complex comprises chiral centers that are not related by a plane or point of symmetry and/or that the ligand or complex comprises an axis of asymmetry due to, for example, restricted rotation, planarity, helicity, molecular knotting or chiral metal complexation.

The term "chiral" refers to molecules which have the property of non superimposability of the mirror image partner.

The term "stereoisomers" refers to compounds which have identical chemical constitution, but differ with regard to the arrangement of the atoms or groups in space.

A "stereoselective process" or an "asymmetric process" is one which produces a particular stereoisomer of a reaction product in preference to other possible stereoisomers of that product.

An "enantioselective reaction" is a reaction that converts an achiral reactant to a chiral, non-racemic product that is enriched in one enantiomer. Enatioselectivity is generally quantified in terms of "enantiomeric excess" ("e. e. "), defined as:

$$e.e. = \left[\frac{(A-B)}{(A+B)}\right] \times 100$$

where A and B are the amounts of enantiomers formed. An enantioselective reaction yields a product with an e.e. greater than zero. Preferred enantioselective reactions yield an e. e. greater than 80%, more preferably greater than 90%, even more preferably greater than 95% and most preferably greater than 98%.

"Ring opening reaction" is recognized in the art and intended to mean a transition-metal catalyzed process in which a nucleophile reacts with a heterocyclic molecule which has at least a double bond, specifically with a double bond situated in position 2 to a heteroatom, and so the pair of electrons of the double bond is displaced, breaking the heteroatom-carbon bond and thus opening the heterocycle.

As mentioned previously, an aspect of the invention is the use of a rhodium-phosphorus complex of formula (I):

$$[Rh(PP)(solv)_2]X \qquad (I)$$

wherein:

PP is a bidentate phophorus ligand or two monodentate phosphorus ligands;
solv is a coordinating solvent; and
X is an anionic counterion,

as catalyst in a ring opening reaction.

Next, the different components of the complex that are advantageously employed in ring opening reactions will be comprehensively described.

*Phosphorus ligand*

Phosphorus ligand represents a ligand covalently bonded to the rhodium by one or two phosphorus atoms. So, both monodentate and bidentate phosphorus ligands are suitable for the present invention. In this sense a "monodentate phosphorus ligand" refers to a molecule containing one phosphorus atom that is covalently bonded to the rhodium, whereas a "bidentate phosphorus ligand" refers to a molecule containing two phosphorus atoms that are covalently bonded to the rhodium. In a preferred embodiment of the invention, the bidentate phosphorus ligand is a diphosphine ligand containing two phosphine groups that are covalently bonded to the rhodium.

The phosphorous ligands used in the present invention are commonly used in organic catalysis by a skilled person. For example, phosphines, phosphinites, phosphonites, phosphites, phosphine-phosphinites, aminophosphines, diaminophosphines are included in the scope of the present invention.

Likewise, both chiral and non-chiral phosphorus ligands are suitable for the present invention.

In a particular embodiment of the invention, the phosphorus ligand is a non-chiral phosphorus ligand. Typical non-chiral phosphorus ligands are $PPh_3$, $P(o\text{-}Tol)_3$, $P(n\text{-}Bu)_3$, $PCy_3$, $P(OEt)_3$, 1,2-bis(diphenylphosphino)ethane (dppe) , 1,4-bis(diphenylphosphino)butane (dppb), 1,1'-bis(diphenylphosphino)ferrocene (dppf).

In another particular embodiment, the phosphorus ligand is a chiral phosphorus ligand, preferably a chiral

bidentate phosphorus ligand, even more preferably a chiral diphosphine ligand. Handbook of Reagents for Organic Synthesis, Chiral Reagents for Asymmetric Synthesis Leo A. Paquette (Wiley; 1 edition (August 15, 2003) covers a broad list of chiral phosphines, which are herein incorporated by reference. Many chiral diphosphine ligands may be purchased from well-known commercial sources such as Sigma Aldrich or Strem.

More preferably, the chiral diphosphine is selected from BPPFA, Ferrophos, FerroTANE, Josiphos, Mandyphos (Ferriphos), Taniaphos, TRAP, Walphos, BICP, Binap, BPE, BPPM, Chiraphos, Deguphos, Diop, DIPAMP, Duphos, Norphos, Pennphos, Phanephos, PPCP, Prophos, Seguphos, and derivatives thereof. These diphosphine ligands are shown in the following scheme:

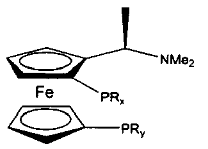

*BPPFA*

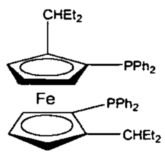

*Ferrophos*

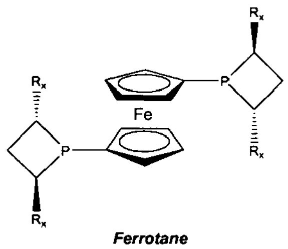

*Ferrotane*

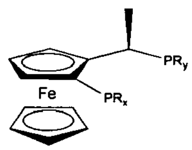

*Josiphos*

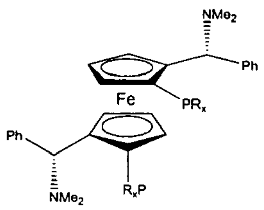

*Mandyphos (Ferriphos)*

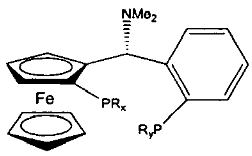

*Taniaphos*

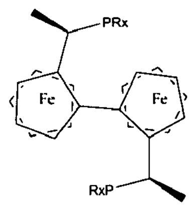

*TRAP*

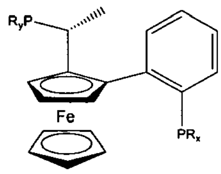

*Walphos*

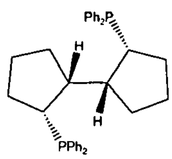

*BICP*

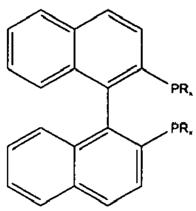

*Binap*

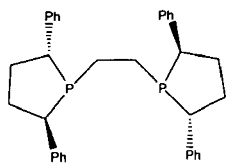

*BPE*

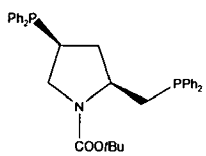

*BPPM*

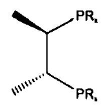

*Chiraphos*

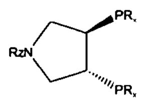

*Deguphos*

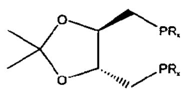

*Diop*

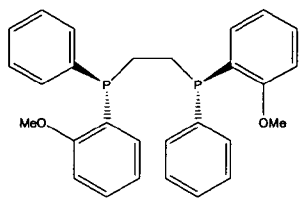

*DIPAMP*

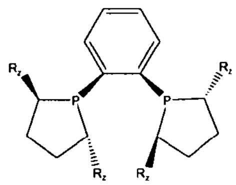

*Duphos*

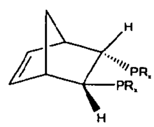

*Norphos*

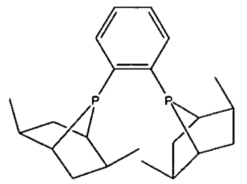
*Pennphos*

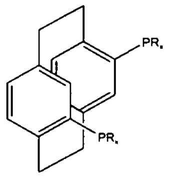
*Phanephos*

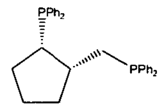
*PPCP*

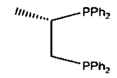
*Prophos*

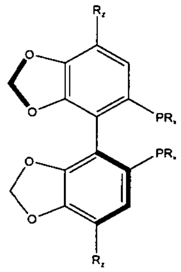
*Segphos*

wherein $R_x$ and $R_y$ are, but not limiting to, substituted or unsubstituted alkyl, such as methyl, ethyl, i-propyl, t-butyl or benzyl; cycloalkyl, such as cyclohexyl; substituted or unsubstituted aryl, such as phenyl, tolyl, 3,5-$(Me)_2$-4-$(MeO)C_6H_2$, 3,5-$(Me)_2C_6H_3$; substituted or unsubstituted heteroaryl, such as 2-furyl.

Examples of these diphosphine ligands include, respectively:

N,N-dimethyl-1-[-2,1'-bis(diphenylphosphino) ferrocenyl] ethylamine);
1,1'-bis(diphenylphosphino)-2,2'-bis(1-ethylpropyl) ferrocene;
1,1'-bis[2,4-diethylphosphetano]ferrocene;
1-[2-(diphenylphosphino)ferrocenyl] ethyldicyclohexyl phosphine;
2,2-bis(N,N-dimethylaminophenylmethyl)-1,1-bis(diphenylphosphino) ferrocene;
[2-diphenylphosphinoferrocenyl](N,N-dimethylamino)(2-diphenylphosphinophenyl) methane;
2.2'-bis[1-(diphenylphophino)ethyl]-1,1'-biferrocene;
1-[2-(2'-diphenylphosphinophenyl)ferrocenyl]ethyldiphenylphosphine;
2,2'-bis(diphenylphosphino)-1,1'-dicyctopentane;
2,2'-bis(diphenylphosphino)-1,1'-binaphthyl;
1,2-bis(dimethylphospholano)ethane;
2-diphenylphosphinomethyl-4-diphenylphosphino-1-t-butoxycarbonylpyrrolidine;
2,3-bis(diphenylphosphino)butane;
1-benzyl-3,4-bis(diphenylphosphino)pyrrolidine;
2,3-O-isopropylidene-2,3-dihydroxy-1,4-bis-(diphenylphosphino)butane;
bis[(2-methoxypheny)phenylphosphino]ethane;
1,2-bis(2,5-dimethylphospholano)benzene;
2,3-bis(diphenylphosphino)-5-norbornene;
1,2-bis(2,5-methyl-7-phosphabicyclo[2.2.1]heptyl)benzene;
4,12-bis(diphenylphosphino)-[2.2]-paracyclophane;
1-(diphenylphosphino)-2-[(diphenylphosphino)methyl]cylopentane;
1,2-bis(diphenylphosphino)propane;
5,5'-bis(diphenylphosphino)-4,4'-bi-1,3-benzodioxole.

In a preferred embodiment of the invention, the diphosphine ligand is a metallocene-type diphosphine ligand. "Metallocene-type diphosphine ligand" means a diphosphine ligand with a metallocene scaffold. A metallocene is an organometallic coordination compound in which one atom of a transition metal is bonded to and only to the face of two cyclopentadienyl [$\eta^5$-$(C_5H_5)$] anions which lie in parallel planes. When the transition metal is iron the metallocene is called ferrocene.

More preferably, the diphosphine ligand is a ferrocene-based diphosphine ligand. In an even preferred embodiment the ferrocene-based diphosphine ligand is selected from the following compounds:

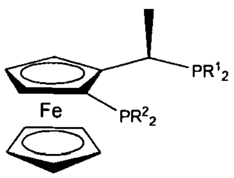

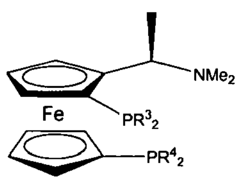

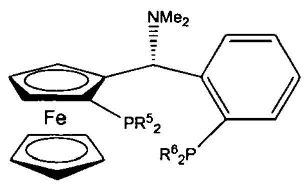

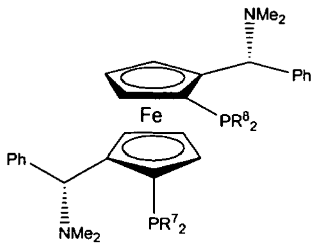

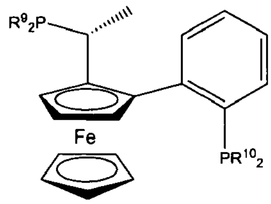

and any stereoisomer, salt or solvate thereof,
wherein

$R^1$ to $R^{10}$ are each independently selected from the group consisting of linear or branched alkyl, sustituted or unsustituted cycloalkyl, sustituted or unsustituted aryl, or substituted or unsubstituted heteroaryl.

Among all the ferrocene-based diphosphine ligands the two following cores are preferred structures:

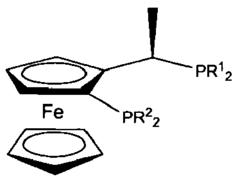

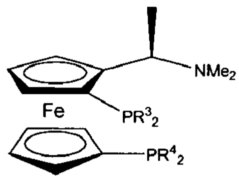

and any stereoisomer, salt or solvate thereof,
wherein $R^1$ to $R^4$ are as defined above for $R^1$ to $R^{10}$.

Even more preferably, the diphosphine ligands are PPF-$P^tBu_2$ and BPPFA.

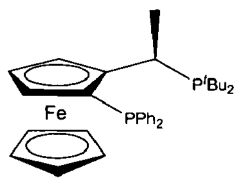

**PPF-$P^tBu_2$**

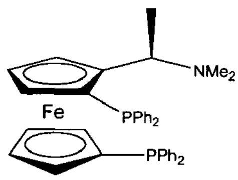

**BPPFA**

and any stereoisomer, salt or solvate thereof.

*Coordinating solvent*

A "coordinating solvent" is one which can act as a ligand forming a covalent bond with a transition metal. Typical coordinating solvents are alkanols and ethers, which have atoms with at least one free electron pair through which they coordinate to the transition metal.

As it will be appreciated, the coordinating solvent in the context of the invention comes from the solvent in which the complex is formed. The coordinating solvent of the rhodium-phosphorus complex of formula (I) is coordinating to the metal by means of an oxygen atom. This solvent is selected from an ether and an alkanol. The ether is preferably selected from tetrahydrofurane, tetrahydropyrane, dioxane, dimethyl ether, diethyl ether, diisopropyl ether, tert-butyl methyl ether and dibutyl ether whereas the alkanol is preferably selected from methanol, ethanol, n-propanol, iso-propanol, n-butanol and tert-butanol. More preferably, the coordinating solvent is tetrahydrofurane or methanol.

*Anionic counterion*

An "anionic counterion" is an ionic species with negative charge that accompanies a cationic transition metal

complex, without coordinating to the metal, in order to maintain electric neutrality.

In a particular embodiment of the invention the anionic counterion is selected from $BF_4^-$, $PF_6^-$, $SbF_6^-$, $AsF_6^-$, $ClO_4^-$, $CH_3SO_3^-$, $CF_3SO_3^-$, $HSO_4^-$, $BPh_4^-$ and B[bis-3,5-trifluoromethyl)phenyl]$_4^-$. Preferably, the anionic counterion is $BF_4^-$.

Accordingly to the above descriptions, preferred rhodium-phosphorus complexes of formula (I) of the invention are selected from [Rh(PPF-P$^t$Bu$_2$)(THF)$_2$]x, [Rh(BPPFA)(THF)$_2$]X, [Rh(PPF-P$^t$BU$_2$)(MeOH)$_2$]X and [Rh(BPPFA)(MeOH)$_2$]X, wherein X is preferably $BF_4$.

<u>Ring opening reaction</u>

The ring opening reaction may be carried out in the presence of a chiral or non-chiral complex, thus leading to an asymmetric or non-asymmetric ring opening reaction, respectively. However, in a preferred embodiment, the ring opening reaction is asymmetric. As stated in the examples below, the process of the invention provides advantageously high enantioselectivities, typically above 98%, and complete conversions, while requiring lower reaction temperatures and shorter reaction times in relation to prior art.

The ring opening involves reacting a $\alpha,\beta$-unsaturated compound of formula (II) and (III):

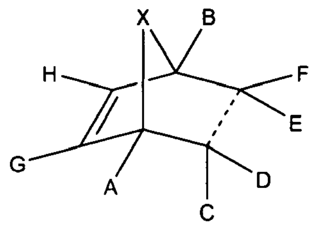

(II)

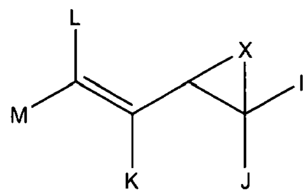

(III)

or a stereoisomer, salt or solvate thereof,

wherein the dotted line represents no bond, a single bond or a double bond;

X is oxygen, sulfur or NR, being R hydrogen, substituted or unsubstituted alkyl, substituted or unsubstituted alkenyl, substituted or unsubstituted aryl or a suitable amino protecting group;

A, B, D, F, G, H, J, K and L are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine;

C and E are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine; or when the dotted line represents a single bond, they can be bound together forming a 5-7 member aliphatic or aromatic ring, optionally substituted; wherein in case C and E form an aromatic ring, D and F do not exist;

J and M are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine; or can be bound together forming the compound:

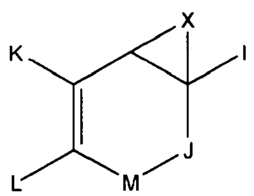

wherein, in this case, J and M are independently selected from substituted or unsubstituted methylene, oxygen, sulfur or NR, being R hydrogen, substituted or unsubstituted alkyl, substituted or unsubstituted alkenyl, substituted or unsubstituted aryl or a suitable amino protecting group; or one of J or M does not exist.

with a nucleophile in the presence of a rhodium-phosphorous complex of formula (I) as defined above.

In a preferred embodiment of the invention X is oxygen or NR, being R hydrogen, substituted or unsubstituted ($C_1$-$C_6$)alkyl, substituted or unsubstituted ($C_1$-$C_6$)alkenyl, substituted or unsubstituted phenyl or being the amino group protected as a carbamate, a sulfonamide or with a silyl group.

In another preferred embodiment of the invention A, B, D, F, G, H, J, K and L are each independently selected from the group consisting of hydrogen, substituted or unsubstituted ($C_1$-$C_6$)alkyl, substituted or unsubstituted ($C_1$-$C_6$) alkenyl, substituted or unsubstituted ($C_5$-$C_6$)cycloalkyl, substituted or unsubstituted phenyl, substituted or unsubstituted heterocyclyl, substituted or unsubstituted ($C_1$-$C_6$)alkoxy, substituted or unsubstituted phenoxy; substituted or unsubstituted ($C_1$-$C_6$)alkylamine; substituted or unsubstituted aniline;

In another preferred embodiment of the invention C and E are each independently selected from the group consisting of hydrogen, substituted or unsubstituted ($C_1$-$C_6$)alkyl, substituted or unsubstituted ($C_1$-$C_6$)alkenyl, substituted or unsubstituted ($C_5$-$C_6$)cycloalkyl, substituted or unsubstituted phenyl, substituted or unsubstituted heterocyclyl, substituted or unsubstituted ($C_1$-$C_6$)alkoxy, substituted or unsubstituted phenoxy; substituted or unsubstituted ($C_1$-$C_6$) alkylamine; substituted or unsubstituted aniline; or when the dotted line represents a single bond, they can be bound together forming a 6 member aliphatic or aromatic ring, optionally substituted; wherein in case C and E form an aromatic ring, D and F do not exist;

In another preferred embodiment of the invention J and M are each independently selected from the group consisting of hydrogen, substituted or unsubstituted ($C_1$-$C_6$)alkyl, substituted or unsubstituted ($C_1$-$C_6$)alkenyl, substituted or unsubstituted ($C_5$-$C_6$)cycloalkyl, substituted or unsubstituted phenyl, substituted or unsubstituted heterocyclyl, substituted or unsubstituted ($C_1$-$C_6$)alkoxy, substituted or unsubstituted phenoxy; substituted or unsubstituted ($C_1$-$C_6$) alkylamine; substituted or unsubstituted aniline; or can be bound together forming the compound:

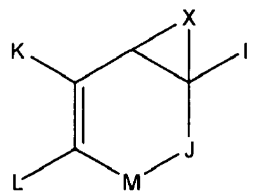

wherein, in this case, J and M are independently selected from substituted or unsubstituted methylene, oxygen, or NR, being R hydrogen, substituted or unsubstituted ($C_1$-$C_6$)alkyl, substituted or unsubstituted ($C_1$-$C_6$)alkenyl, substituted or unsubstituted phenyl or being the amino group protected as a carbamate, a sulfonamide or with a silyl group; or one of J or M does not exist.

*Nucleophile*

In the context of the present invention, the term "nucleophile" refers to a reagent that forms a chemical bond to its reaction partner (the electrophile) by donating both bonding electrons. Both neutral and anionic nucleophiles are considered in the present invention [for references related to nucleophilicity, please see: Phan T. B.; Breugst, M.; Mayr, H. Angew. Chem. Int. Ed. 2006, 45, 3869-3874. Mayr, H.; Patz, M. Angew. Chem. Int. Ed. Engl. 1994, 33, 938-957].

Non-limiting examples of nucleophiles used in this process are for instance an halogen; a carbon nucleophile selected from 3-indol and activated methylene group; a boronic acid; an oxygen nucleophile selected from water, an alcohol, an ether and a carboxylate; a nitrogen nucleophile selected from ammonia, an amine, an azide, cyanide, isocyanate and isothiocyanate; a sulphur nucleophile selected from a thiol and a thioether; selenocyanate or a phosphine.

Activated methylene groups have electron withdrawing groups in the $\alpha$-position, such as carbonyl or ester groups, such as in acetoacetates.

Preferred nucleophiles are alcohols, ethers and amines.

*Reaction Solvent*

The ring opening reaction is advantageously carried out in the presence of a solvent selected from an ether, an alcohol, a ketone, an ester, an amine, a chlorine-containing solvent, an aromatic solvent, an aprotic polar solvent and mixtures thereof.

In a particular embodiment of the invention the solvent is selected from tetrahydrofurane, tetrahydropyrane,

dioxane, dimethyl ether, diethyl ether, dipropyl ether, diisopropyl ether, dibutyl ether, methyl tert-butyl ether, dibenzyl ether, anisol, triethylamine, methanol, ethanol, propanol, isopropanol, butanol, tert-butanol, acetone, ethyl acetate, triethylamine, piperidine, pyridine, tetrachloromethane, dichloromethane, chloroform, 1,2-dichloroethane, benzene, toluene, xylene, dimethylformamide, dimethylacetamide, dimethylsulfoxide, acetonitrile, benzonitrile, nitromethane, propylene carbonate or mixtures thereof.

In another particular embodiment the solvent of the ring opening reaction is also the nucleophile.

In a particular embodiment of the invention, the α,β-unsaturated compound is an alkene of formula (IIa):

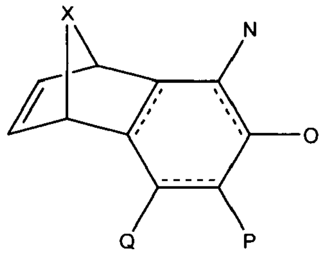

(IIa)

wherein 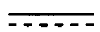 represents a single bond or a double bond,
X is oxygen, sulfur or NR, being R hydrogen, substituted or unsubstituted alkyl, substituted or unsubstituted alkenyl, substituted or unsubstituted aryl or a suitable amino protecting group;
N, O, P and Q each independently are selected from hydrogen, a substituted or unsubstituted alkyl, a substituted or unsubstituted alkenyl, a substituted or unsubstituted cycloalkyl, a substituted or unsubstituted aryl, a substituted or unsubstituted alkoxy, substituted or unsubstituted aryloxy, substituted or unsubstituted alkylamine, substituted or unsubstituted arylamine, halogen and nitro.

More preferably, the α,β-unsaturated compound is an alkene of formula (IIa) wherein N, O, P and Q are independently hydrogen, methyl, methoxy and halogen.

Another aspect of the present invention is directed to a rhodium-phosphorus complex of the formula (I'):

$[Rh(PP')(solv)_2]X$ (I')

wherein

PP' is a metallocene-type diphosphine ligand,
solv is a coordinating solvent, and
X is an anionic counterion,

with the proviso that $[Rh(PPF\text{-}PCy_2)(MeOH)_2]BF_4$ is not included.

The solvent (solv) and the conterion (X) have the meaning previously defined for the complex of formula (I), whereas PP' is a metallocene-type diphosphine ligand.

In a particular embodiment, the metallocene-type diphosphine ligand is preferably a ferrocene-based diphosphine ligand. According to this definition, the ferrocene-based diphosphine ligand is selected from the following compounds:

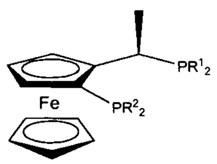

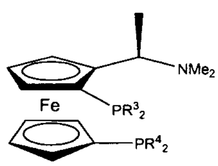

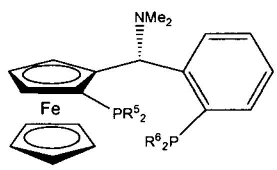

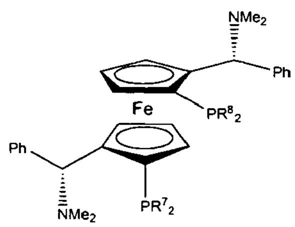

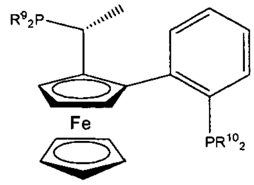

and any stereoisomer, salt or solvate thereof,
wherein

$R^1$ to $R^{10}$ are each independently selected from the group consisting of linear or branched alkyl, sustituted or unsustituted cycloalkyl, sustituted or unsustituted aryl, or substituted or unsubstituted heteroaryl.

As stated above, among all the ferrocene-based diphosphine ligands, the two following compounds are preferred structures:

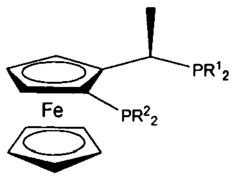

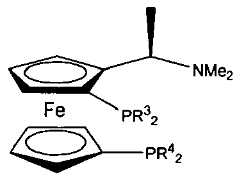

and any stereoisomer, salt or solvate thereof,
wherein $R^1$ to $R^4$ are as defined above.

Even more preferably, the diphosphine ligand is selected from PPF-$P^tBU_2$ and BPPFA.

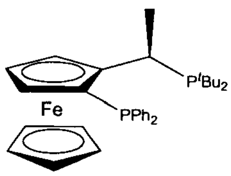

**PPF-$P^tBu_2$**

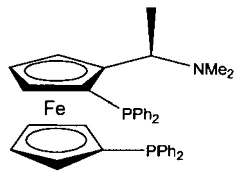

**BPPFA**

or a stereoisomer, salt or solvate thereof,

Likewise, preferred rhodium-phosphorus complexes of formula (I') of the invention are selected from [Rh(PPF-$P^tBu_2$)$(THF)_2$]X, [Rh(BPPFA)$(THF)_2$]X, [Rh(PPF-$P^tBu_2$)$(MeOH)_2$]X and [Rh(BPPFA)$(MeOH)_2$]X, wherein X preferably is $BF_4$.

<u>Preparation of rhodium-phosphorus complex (I')</u>

In another aspect, the present invention refers to a process for the preparation of a rhodium-phosphorus complex of formula (I') as defined above, which comprises the hydrogenation of a rhodium diolefin complex of formula (IV) or a rhodium mono-olefin complex of formula (V) in the presence of a suitable coordinating solvent (solv),

[Rh(PP')(diolefin)]X (IV)

[M(PP')$(mono\text{-}olefin)_2$]X (V)

wherein PP', X and (solv) have the meanings as defined above for the complex of formula (I').

In a particular embodiment, the diolefin is selected from the group consisting of 1,3-cyclooctadiene, 1,4-cyclooctadiene, 1,5-cyclooctadiene (COD), 2,5-norbomadiene (NBD), 1,5-hexadiene and 1,6-heptadiene. In another particular embodiment, the mono-olefin is selected from ethylene, hexane and octene.

The suitable coordinating solvent is incorporated to the complex displacing the diolefin or mono-olefin after the hydrogenation thereof.

In a particular embodiment, once the rhodium-phosphorus complex is obtained, said process further comprises the subsequent addition of a compound of formula (II) or (III) as defined above and a nucleophile to promote the ring opening reaction of said compound of formula (II) or (III).

Tyipical nucleophiles for this process are alcohols, phenols, amines, and stabilized carbanions such as malonates and derivatives.

In a preferred embodiment, the nucleophile is an alcohol or an amine, preferably is methanol or dimethylamine.

In a preferred embodiment, the compound of formula (II) is a compound of formula (IIa'):

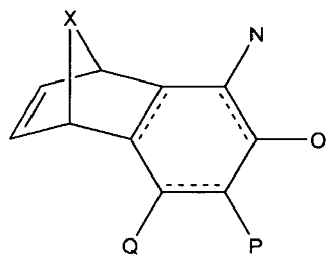

(IIa')

wherein N, O, P and Q are selected from hydrogen, a substituted or unsubstituted alkyl, a substituted or unsubstituted alkenyl, a substituted or unsubstituted cycloalkyl, a substituted or unsubstituted aryl, a substituted or unsubstituted alkoxy, a substituted or unsubstituted aryloxy, substituted or unsubstituted alkylamine, substituted or unsubstituted arylamine, halogen and nitro.

In a more preferred embodiment, the compound of formula (IIa') is that wherein N is hydrogen, methyl, methoxy or halogen, and O, P and Q are hydrogen.

As mentioned above, the ring opening reaction can be asymmetric or non-asymmetric depending on the presence or absence of chirality in the rhodium complex used in the reaction. However, in the context of the present invention, it is particularly preferred the execution of an asymmetric ring opening reaction.

A simplified version of the proposed asymmetric catalytic pathway for this transformation is the following: firstly, the chiral rhodium complex binds to the heteroatom and the alkene; afterwards, oxidative insertion of rhodium catalyst to carbon-heteroatom bond and an $S_N2'$ displacement of the rhodium catalyst by the nucleophile gives the product and regenerates the catalyst. Nucleophilic attack with inversion provides the product in an $S_N2'$ fashion relative to the metal.

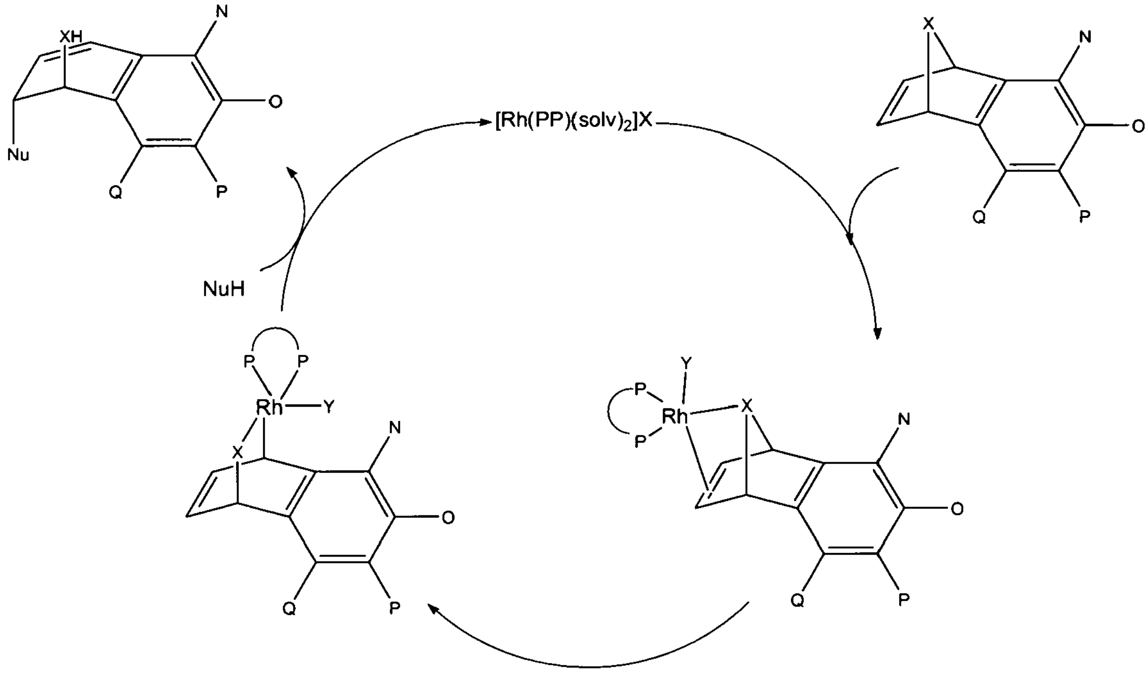


In a particular embodiment, the product obtained after the asymmetric ring opening reaction takes place is selected from:

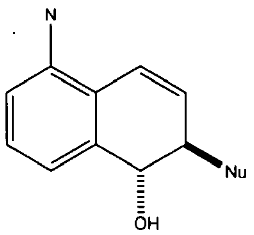
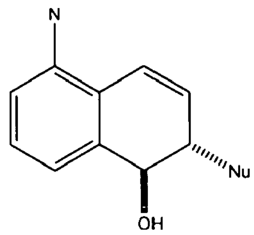
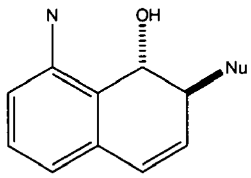
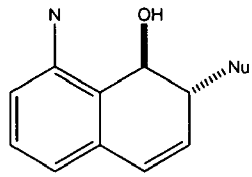

wherein N is hydrogen, methyl, methoxy or halogen; and

Nu is a nucleophile selected from an alcohol or an amine, preferably is methanol, dimethylamine or monomethylamine

Finally, another aspect of the present invention describes a rhodium-phosphorus complex (I') obtainable by the process which comprises the hydrogenation of a metal diolefin complex of formula (IV) or a metal mono-olefin complex of formula (V) in the presence of a suitable coordinating solvent (solv),

[Rh(PP')(diolefin)]X (IV)

[M(PP')(mono-olefin)$_2$]X (V)

wherein PP', X and (solv) have the meanings as defined above for the complex of formula (I').

The following non-limiting examples will further illustrate specific embodiments of the invention.

## EXAMPLES

<u>**Example 1** - Synthesis of rhodium-phosphorus complexes</u>

**PPF-P$^t$Bu$_2$**

[Rh((*S*,*R*)-PPF-P$^t$Bu$_2$)(NBD)]BF$_4$ or [Rh((*S*,*R*)-PPF-P$^t$Bu$_2$)(COD)]BF$_4$ (0.01 mmol) is dissolved in 3 mL of THF-d$_8$ or MeOH-d$_4$ under argon atmosphere. Hydrogen is pressed on the solution, which is then allowed to stir under hydrogen atmosphere for ca. 5 min.

- **[Rh((*S,R*)-PPF-$P^tBu_2$)($MeOH)_2$]$BF_4$**
  $^1$H-NMR: 8.59-8.51 (2H, m); 7.67-7.56 (5H, m); 7.46-7.39 (3H, m); 4.96-4.89 (m); 4.63 (1H, br. s); 4.35 (1H, br. s); 4.17 (1 H, br. s); 3.81-3.74 (5H, m); 3.39-3.31 (m); 2.88-2.82 (1 H, m); 2.00-1.95 (3H, m); 1.71-1.66 (10H, m); 1.34-1.28 (10H, m).
  $^{31}$P-NMR (in MeOH-$d_4$): 112.3 (J=213.2/54.7 Hz); 49.6 (J=211.5/54.6 Hz)

- **[Rh((*S,R*)-PPF-$P^tBu_2$)$(THF)_2$]$BF_4$**
  $^1$H-NMR: signals (except for arene protons) covered by solvent signals $^{31}$P-NMR (in THF-$d_8$): 113.2 (J=206.7/54.7Hz); 51.0 (J=230.2/53.9Hz)

**DPPF:**

[Rh(DPPF)(NBD)]$BF_4$ or [Rh(DPPF)(COD)]$BF_4$ (0.01 mmol) is dissolved in 3 mL of MeOH-$d_4$ under argon atmosphere. Hydrogen is pressed on the solution, which is then allowed to stir under hydrogen atmosphere for ca. 5 and 45 min, respectively.

- **[Rh(DPPF)$(MeOH)_2$]$BF_4$**
  $^1$H-NMR (in MeOH-$d_4$): 7.96-7.89 (8H, m); 7.55-7.41 (12H, m); 4.90 (s); 4.39-4.37 (4H, m); 4.29-4.26 (4H, m); 3.33-3.31 (m). (in NMR also signals of norbomadiene)
  $^{31}$P-NMR (in MeOH-$d_4$): 54.9 (213.7 Hz).

<u>**Example 2 -** Experimental procedure of ring opening</u>

[Rh((*S*,*R*)-PPF-$P^tBu_2$)(NBD)]BF4 (0.01 mmol) is dissolved in 3 mL of THF under argon atmosphere. Hydrogen is pressed on the solution, which is then allowed to stir under hydrogen atmosphere for ca. 5 min. Hydrogen is exchanged by argon by freezing the solution and securating the gas phase above the solution with argon. This procedure is repeated 3 times. To the cold solvent complex a solution of the substrate (1 mmol) dissolved in 3 ml of THF is added via cannula. The nucleophile (1 mmol) is added to the cool substrate complex solution a) directly via syringe (in case of liquids) or b) as a THF (ca. 4ml) solution via cannula from a separate flask (in case of solids). The reaction mixture was then heated at 50°C until the reaction was finished (as judged by TLC or determined separately by HPLC). The solvent was then removed in vacuo and the resulting mixture purified by flash chromatography.

For comparative purposes, other diphosphine complexes of the art have also been tested in the asymmetric ring opening reaction of oxobenzonorbomadiene.

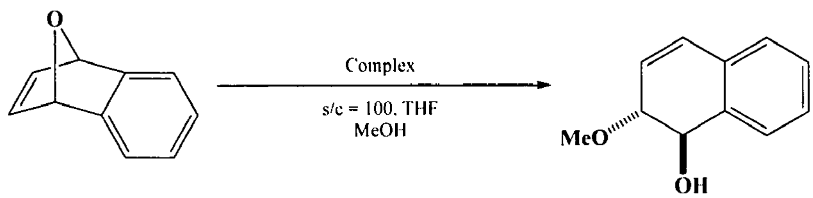


| Complex | s/nu | Temp(°C) | Time(min) | Yield(conv.)(%) | ee(%) |
|---|---|---|---|---|---|
| [Rh(COD)Cl]$_2$/((*S*,*R*)-PPF-$P^tBu_2$) | 1:7 | 80 | 900 | 96 | 97 |
| [Rh((*S*,*R*)-PPF- | 1:7 | 50 | 70 | 90(100) | 98.8 |
| $P^tBu_2$)$(THF)_2$]$BF_4$ | **1:1** | **50** | **35** | **(100)** | **98.8** |
| s/c = substrate/catalyst ratio; s/nu = substrate/nucleophile ratio; yield expressed as isolated yield (conversion yield in brackets) | | | | | |

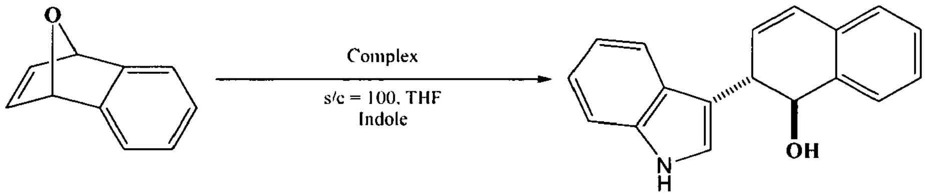


| Complex | s/nu | Temp(°C) | Time(min) | Yield(conv.)(%) | ee(%) |
|---|---|---|---|---|---|
| [RhI((*S*,*R*)-PPF-P$^t$Bu$_2$)] | 1:5 | 80 | 30 | 93 | 97 |
| [Rh((*S*,*R*)-PPF-P$^t$Bu$_2$) | 1:5 | 50 | 70 | (100) | 98.2 |
| (THF)2]BF$_4$ | **1:1** | **50** | **50** | **(100)** | **98.9** |
| s/c = substrate/catalyst ratio; s/nu = substrate/nucleophile ratio; yield expressed as isolated yield (conversion yield in brackets) | | | | | |

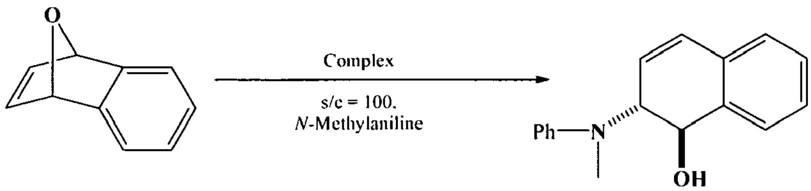


| Complex | s/nu | Temp(°C) | Time(min) | Yield(conv.)(%) | ee(%) |
|---|---|---|---|---|---|
| [RhI((*S*,*R*)-PPF-P$^t$Bu$_2$)] | 1:5 | 80 | 120 | 96 | 92 |
| [Rh((*S*,*R*)-PPF-P$^t$Bu$_2$) | 1:5 | 50 | 45 | (100) | 98.0 |
| (THF)$_2$]BF$_4$ | **1:1** | **50** | **35** | **(100)** | **98.0** |
| s/c = substrate/catalyst ratio; s/nu = substrate/nucleophile ratio; yield expressed as isolated yield (conversion yield in brackets) | | | | | |

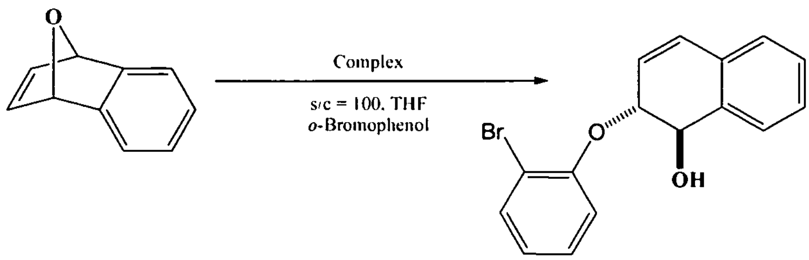


| Complex | s/nu | Temp(°C) | Time(min) | Yield(conv.)(%) | ee(%) |
|---|---|---|---|---|---|
| [RhI((*S*,*R*)-PPF-P$^t$Bu$_2$)] | 1:5 | 80 | 90 | 94 | 95 |
| [Rh((*S*,*R*)-PPF-P$^t$Bu$_2$) | 1:5 | 50 | 55 | (100) | 98.5 |

(continued)

| Complex | s/nu | Temp(°C) | Time(min) | Yield(conv.)(%) | ee(%) |
|---|---|---|---|---|---|
| $(THF)_2]BF_4$ | **1:1** | **50** | **40** | **(100)** | **99.3** |
| s/c = substrate/catalyst ratio; s/nu = substrate/nucleophile ratio; yield expressed as isolated yield (conversion yield in brackets) | | | | | |

As it is shown, the rhodium solvent complex of the invention provides better results than the complex used in the prior art. Specifically, the transformation runs at lower temperatures and in less than one hour. Also, there is no need of using large amount of nucleophile, since the reaction takes place with complete conversions and excellent enantioselectivities with only one equivalent of nucleophile.

## Claims

1. Use of a rhodium-phosphorus complex of formula (I):

$$[Rh(PP)(solv)_2]X \qquad (I)$$

wherein:

PP is a bidentate phosphorus ligand or two monodentate phosphorus ligands;
solv is a coordinating solvent; and
X is an anionic counterion,
as catalyst in a ring opening reaction.

2. Use according to claim 1 wherein the phosphorus ligand is a chiral phosphorus ligand.

3. Use according to claim 2 wherein the chiral phosphorus ligand is a chiral bidentate ligand, preferably a chiral diphosphine ligand.

4. Use according to claim 3 wherein the chiral diphosphine ligand is selected from the group consisting of BPPFA, Ferrophos, FerroTANE, Josiphos, Mandyphos (Ferriphos), Taniaphos, TRAP, Walphos, BICP, Binap, BPE, BPPM, Chiraphos, Deguphos, Diop, DIPAMP, Duphos, Norphos, Pennphos, Phanephos, PPCP, Prophos, Seguphos and derivatives thereof.

5. Use according to claim 3 wherein the diphosphine ligand is a metallocene-type diphosphine ligand.

6. Use according to claim 5 wherein the metallocene-type diphosphine ligand is a ferrocene-based diphosphine ligand.

7. Use according to claim 6 wherein the ferrocene-based diphosphine ligand is selected from the following compounds:

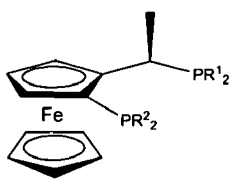 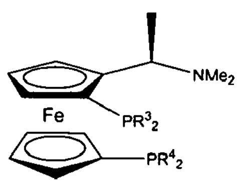 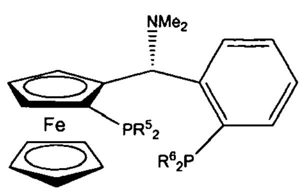

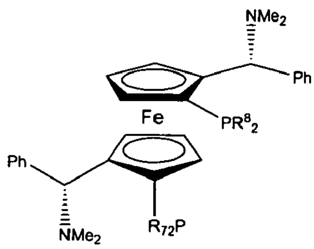
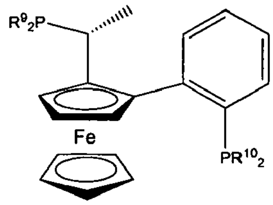

and any stereoisomer, salt or solvate thereof,
wherein
$R^1$ to $R^{10}$ are each independently selected from the group consisting of linear or branched alkyl, sustituted or unsustituted cycloalkyl, sustituted or unsustituted aryl, or substituted or unsubstituted heteroaryl.

**8.** Use according to claim 7 wherein the ferrocene-based diphosphine ligand is selected from:

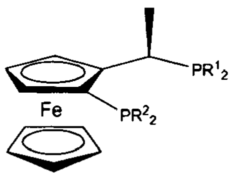
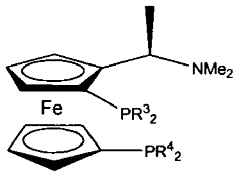

and any stereoisomer, salt or solvate thereof,
wherein $R^1$ to $R^4$ are as defined in claim 7.

**9.** Use according to claim 8 wherein the ferrocene-based diphosphine ligand is selected from:

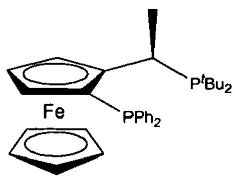
**PPF-P^tBu_2**

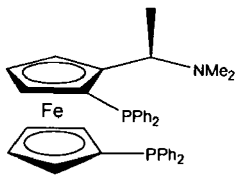
**BPPFA**

and any stereoisomer, salt or solvate thereof.

**10.** Use according to any of claims 1 to 9 wherein the coordinating solvent is selected from an ether and an alkanol.

**11.** Use according to claim 10 wherein the ether is selected from tetrahydrofurane, tetrahydropyrane, dioxane, dimethyl ether, diethyl ether, diisopropil ether, methyl tert-butyl ether and dibutyl ether, and the alkanol is selected from methanol, ethanol, n-propanol, iso-propanol, n-butanol and tert-butanol.

**12.** Use according to any of claims 1 to 11 wherein the anionic counterion is selected from $BF_4^-$, $PF_6^-$, $SbF_6^-$, $AsF_6^-$, $ClO_4^-$, $CH_3SO_3^-$, $CF_3SO_3^-$, $HSO_4^-$, $BPh_4^-$ and B[bis-3,5-trifluoromethyl)phenyl]$_4^-$.

**13.** Use according to any of claims 1 to 12 wherein the rhodium-phosphorous complex of formula (I) is selected from [Rh(PPF-P$^t$Bu$_2$)(THF)$_2$]X, [Rh(BPPFA)(THF)$_2$]X, [Rh(PPF-P$^t$Bu$_2$)(MeOH)$_2$]X and [Rh(BPPFA)(MeOH)$_2$]X.

**14.** Use according to claim 13 wherein the anionic counterion X is $BF_4^-$.

**15.** A process for the catalytic ring opening of $\alpha,\beta$-unsaturated compounds of formula (II) and (III):

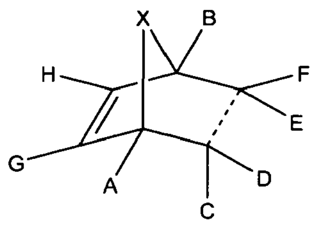

(II)

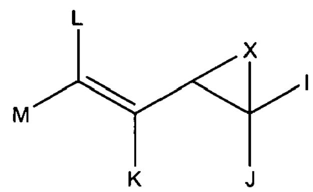

(III)

or a stereoisomer, salt or solvate thereof,
wherein the dotted line represents no bond, a single bond or a double bond;
X is oxygen, sulfur or NR, being R hydrogen, substituted or unsubstituted alkyl, substituted or unsubstituted alkenyl, substituted or unsubstituted aryl or a suitable amino protecting group;
A, B, D, F, G, H, J, K and L are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine;
C and E are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine; or when the dotted line represents a single bond, they can be bound together forming a 5-7 member aliphatic or aromatic ring, optionally substituted; wherein in case C and E form an aromatic ring, D and F do not exist;
J and M are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine; or can be bound together forming the compound:

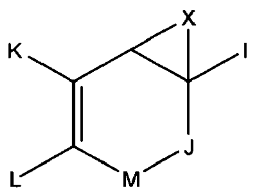

wherein, in this case, J and M are independently selected from substituted or unsubstituted methylene, oxygen, sulfur or NR, being R hydrogen, substituted or unsubstituted alkyl, substituted or unsubstituted alkenyl, substituted or unsubstituted aryl or a suitable amino protecting group; or one of J or M does not exist;
in the presence of a rhodium-phosphorus complex of formula (I) as defined in any of claims 1 to 14.

**16.** The process according to claim 15 wherein the compound of formula (II) is a heterobicyclic alkene of formula (IIa):

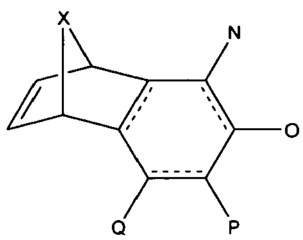

(IIa)

wherein represents a single bond or a double bond,
X is oxygen, sulfur or NR, being R hydrogen, substituted or unsubstituted alkyl, substituted or unsubstituted alkenyl, substituted or unsubstituted aryl or a suitable amino protecting group;
N, O, P and Q each independently are selected from hydrogen, a substituted or unsubstituted alkyl, a substituted or unsubstituted alkenyl, a substituted or unsubstituted cycloalkyl, a substituted or unsubstituted aryl, a substituted or unsubstituted alkoxy, substituted or unsubstituted aryloxy, substituted or unsubstituted alkylamine, substituted or unsubstituted arylamine, halogen and nitro.

**17.** The process according to claim 16 wherein N, O, P and Q are independently hydrogen, methyl, methoxy and halogen.

**18.** A rhodium-phosphorus complex of the formula (I'):

$$[Rh(PP')(solv)_2]X \qquad (I')$$

wherein

PP' is a metallocene-type diphosphine ligand,
solv is a coordinating solvent, and
X is an anionic counterion.

with the proviso that $[Rh(PPF\text{-}PCy_2)(MeOH)_2]BF_4$ is not included.

**19.** The rhodium-phosphorus complex according to claim 18, wherein the metallocene-type diphosphine ligand is a ferrocene-based diphosphine ligand.

**20.** The rhodium-phosphorus complex according to claim 19, wherein the ferrocene-based diphosphine ligand is selected from the group consisting of the following compounds:

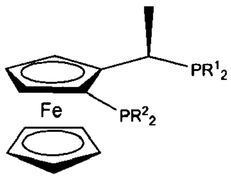
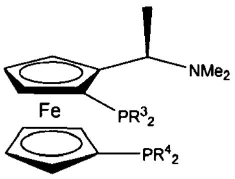
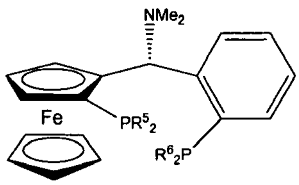

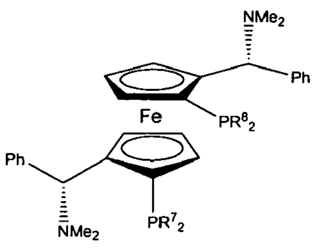
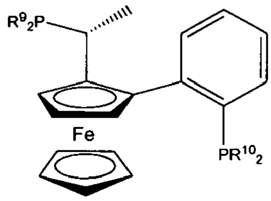

and any stereoisomer, salt or solvate thereof,
wherein
$R^1$ to $R^{10}$ are each independently selected from the group consisting of branched alkyl, preferably tert-butyl, cyclohexyl, sustituted or unsustituted aryl, or substituted or unsubstituted heteroaryl.

**21.** The rhodium-phosphorus complex according to claim 20, wherein ferrocene-based diphosphine ligand is selected from:

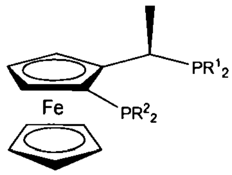 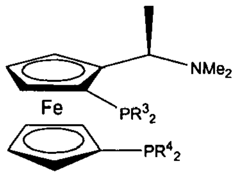

and any stereoisomer, salt or solvate thereof, wherein $R^1$ to $R^4$ are as defined in claim 20.

**22.** The rhodium-phosphorus complex according to claim 21 wherein $R^1$, $R^3$ and $R^4$ are phenyl and $R^2$ is a branched alkyl, preferably is tert-butyl.

**23.** The rhodium-phosphorus complex according to claims 18 to 22, wherein the anionic counterion X is selected from the group consisting of $BF_4^-$, $PF_6^-$, $SbF_6^-$, $AsF_6^-$, $ClO_4^-$, $CH_3SO_3^-$, $CF_3SO_3^-$, $HSO_4^-$, $BPh_4^-$ and B[bis-3,5-trifluoromethyl)phenyl]$_4$.

**24.** The rhodium-phosphorus complex according to claims 18 to 23 wherein the coordinating solvent is selected from an ether and an alkanol.

**25.** The rhodium-phosphorus complex according to claim 24 wherein the ether is selected from tetrahydrofurane, tetrahydropyrane, dioxane, dimethyl ether, diethyl ether, diisopropyl ether, methyl tert-butyl ether and dibutyl ether, and the alkanol is selected from methanol, ethanol, n-propanol, iso-propanol, n-butanol and tert-butanol.

**26.** The rhodium-phosphorus complex according to any of claims 18 to 25 which is selected from [Rh(PPF-P$^t$Bu$_2$)(THF)$_2$]X, [Rh(BPPFA)(THF)$_2$]X, [Rh(PPF-P$^t$Bu$_2$)(MeOH)$_2$]X and [Rh(BPPFA)(MeOH)$_2$]X.

**27.** The rhodium-phosphorus complex according to claim 26 wherein the anionic counterion X is $BF_4^-$.

**28.** A process for the preparation of a rhodium-phosphorus complex as defined in any of claims 18 to 27, which comprises the hydrogenation of a metal diolefin complex of formula (IV) or a metal mono-olefin complex of formula (V) in the presence of a suitable coordinating solvent (solv),

[Rh(PP')(diolefin)]X (IV)

[M(PP')(mono-olefin)$_2$]X (V)

wherein PP', X and (solv) have the meanings as defined above for the complex of formula (I').

**29.** The process according to claim 28, wherein the diolefin is selected from the group consisting of 1,3-cyclooctadiene, 1,4-cyclooctadiene, 1,5-cyclooctadiene (COD), 2,5-norbomadiene (NBD), 1,5-hexadiene and 1,6-heptadiene and the mono-olefin from ethylene, hexene and octene.

**30.** The process according to claim 28 or 29, wherein the suitable coordinating solvent is as defined in claims 24 and 25.

**31.** The process according to any of claims 28 to 30 which further comprises the subsequent addition of a compound of formula (II) or (III) as defined in claim 15 and a nucleophile to promote the ring opening reaction of said compound of formula (11) or (III).

**32.** The process according to claim 31 wherein the compound of formula (II) is a compound of formula (IIa'):

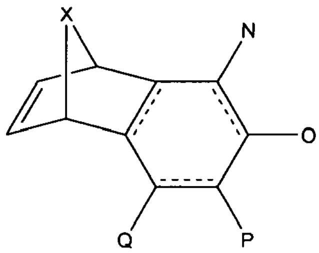

(IIa')

wherein N, O, P and Q are selected from hydrogen, a substituted or unsubstituted alkyl, a substituted or unsubstituted alkenyl, a substituted or unsubstituted cycloalkyl, a substituted or unsubstituted aryl, a substituted or unsubstituted alkoxy, a substituted or unsubstituted aryloxy, substituted or unsubstituted alkylamine, substituted or unsubstituted arylamine, halogen and nitro.

**33.** The process according to claim 32 wherein N is hydrogen, methyl, methoxy or halogen, and O, P and Q are hydrogen.

**34.** The process according to any of claims 31 to 33 wherein the nucleophile is an alcohol or an amine, preferably is methanol or dimethylamine.

**35.** The process according to any of claims 31 to 34 wherein the obtained product is selected from:

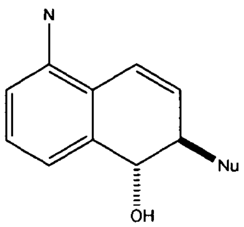
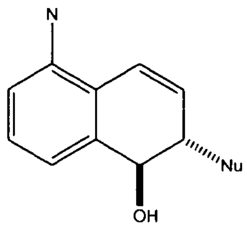
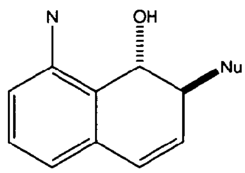
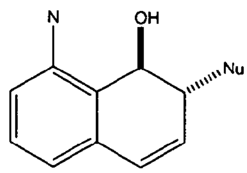

wherein N is hydrogen, methyl, methoxy or halogen; and
Nu is a nucleophile selected from an alcohol or an amine, preferably is methanol, dimethylamine or monomethylamine.

**36.** A metal-diphosphine complex obtainable by the process as defined in any of claims 28 to 30.

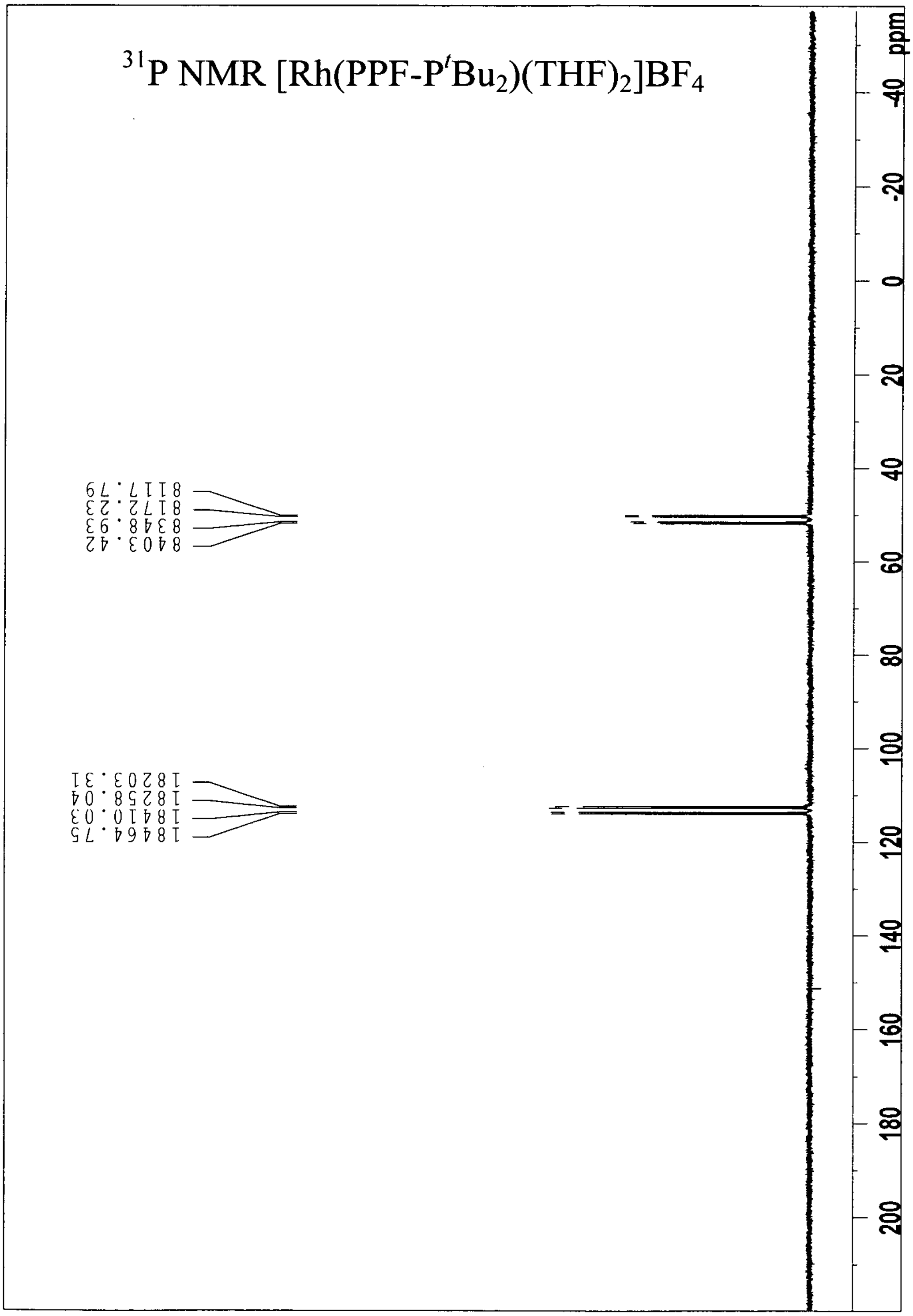
31P NMR [Rh(PPF-PtBu2)(THF)2]BF4
8117.79
8172.23
8348.93
8403.42
18203.31
18258.04
18410.03
18464.75
ppm

**Fig. 1**

European Patent Office

# EUROPEAN SEARCH REPORT

Application Number
EP 07 38 0349

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ALLGEIER, SLONE, MIRKIN, LIABLE-SANDS, YAP, RHEINGOLD: "Electrochemical controlling ligand binding affinity for transition metals via RHLs: The importance of electrostatic effects" JOURNAL OF THE AMERICAN CHEMICAL SOCIETY, vol. 119, 1997, pages 550-559, XP002478840 * compounds 12,13 * | 18,19, 23,27,36 | INV. C07C41/03 C07C209/18 C07D209/12 C07F15/00 |
| X | WO 2007/123957 A (DOW GLOBAL TECHNOLOGIES INC [US]; JACKSON PHILIP M [GB]; LENNON IAN CA) 1 November 2007 (2007-11-01) Compound of example 11 treated with MeOH and hydrogen; table (iv) | 18-30,36 | |
| X | WO 2006/108562 A (DSM IP ASSETS BV [NL]; BONRATH WERNER [DE]; KARGE REINHARD [DE]; ROESS) 19 October 2006 (2006-10-19) * example 22 * | 18-30,36 | |
| X | BARBARO, BIANCHINI, GIAMBASTIANI, PARISEL: "Progress in stereoselective catalysis by metal complexes with chiral ferrocenyl phosphines" COORDINATION CHEMISTRY REVIEWS, vol. 248, 2004, pages 2131-2150, XP002478841 * page 2133, left-hand column, lines 5-7 * * tables 2,entry,5 * * table 5 * * tables 7,entries,2-7 * * tables 8,entries,1,and,5 * * page 2137, right-hand column, lines 3-5 * * tables 11,entry,3 * | 18-30,36 | TECHNICAL FIELDS SEARCHED (IPC) C07C C07D C07F |

-/--

The present search report has been drawn up for all claims

| Place of search | Date of completion of the search | Examiner |
|---|---|---|
| Munich | 5 May 2008 | Pérez Carlón, Raquel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

European Patent Office

# EUROPEAN SEARCH REPORT

Application Number
EP 07 38 0349

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YOU, DREXLER, ZHANG, FISCHER, HELLER: "Preparation and asymmetric hydrogenation of beta-aryl substituted beta-acylaminoacrylates" ANGEWANDTE CHEMIE INTERNATIONAL EDITION (ENGLISH), vol. 42, 2003, pages 913-916, XP009099507 * tables 1,entries,5-8 * | 18-30,36 | |
| X | TARAROV, KADYROV, MONSEES, RIERMEIER, BÖRNER: "Asymmetric cleavage of racemic 1,3-oxazolidines - A new dynamic process in homogeneous Rh(I)-catalyzed hydrogenation" ADVANCED SYNTHESIS & CATALYSIS, vol. 345, 2003, pages 239-245, XP009099515 Synthesis of 2 from 1 | 1-4, 10-12 | |
| A | HOLZ J ET AL: "Cooperative attractive interactions in asymmetric hydrogenations with dihydroxydiphosphine Rh(I) catalysts - a competition study" JOURNAL OF ORGANOMETALLIC CHEMISTRY, ELSEVIER-SEQUOIA S.A. LAUSANNE, CH, vol. 603, no. 1, 22 May 2000 (2000-05-22), pages 61-68, XP004213439 ISSN: 0022-328X * page 63, right-hand column, lines 14-22 * | 1-36 | TECHNICAL FIELDS SEARCHED (IPC) |

-/--

The present search report has been drawn up for all claims

| Place of search | Date of completion of the search | Examiner |
|---|---|---|
| Munich | 5 May 2008 | Pérez Carlón, Raquel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

2

European Patent Office

# EUROPEAN SEARCH REPORT

Application Number
EP 07 38 0349

## DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| D,A | LAUTENS MARK ET AL: "Transition metal-catalyzed enantioselective ring-opening reactions of oxabicyclic alkenes" ACCOUNTS OF CHEMICAL RESEARCH, ACS, WASHINGTON, DC, US, vol. 36, no. 1, 1 January 2003 (2003-01-01), pages 48-58, XP009090288 ISSN: 0001-4842 Rhodium-catalyzed asymmetric ring opening with heteroatom nucleophiles ----- | 1-36 | |
| A | LAUTENS, FAGNOU: "Rhodium-catalyzed asymmetric ring opening reactions of oxabicyclic alkenes: Catalyst and substrate studies leading to a mechanistic working model" PNAS, vol. 101, 2004, pages 5455-5460, XP002478843 * the whole document * ----- | 1-36 | TECHNICAL FIELDS SEARCHED (IPC) |

The present search report has been drawn up for all claims

| Place of search | Date of completion of the search | Examiner |
| --- | --- | --- |
| Munich | 5 May 2008 | Pérez Carlón, Raquel |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 07 38 0349

This annex lists the patent family members relating to the patent documents cited in the above-mentioned European search report.
The members are as contained in the European Patent Office EDP file on
The European Patent Office is in no way liable for these particulars which are merely given for the purpose of information.

05-05-2008

| Patent document cited in search report | Publication date | Patent family member(s) | Publication date |
|---|---|---|---|
| WO 2007123957 A | 01-11-2007 | NONE | |
| WO 2006108562 A | 19-10-2006 | KR 20070118110 A | 13-12-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2001030734 A, Fagnou, K.; Lautens, M.
- EP 1225166 A

### Non-patent literature cited in the description


- **LAUTENS, M. ; ROVIS, T. J.** *Org. Chem.,* 1997, vol. 62, 5246-5247
- **LAUTENS, M. ; ROVIS, T.** *Tetrahedron,* 1999, 8967-8976
- **LAUTENS, M. ; COLUCCI, J. T. ; HIEBERT, S. ; SMITH, N. D. ; BOUCHAIN, G.** *Org. Lett.,* 2002, vol. 4, 1879-1882
- **LAUTENS, M. ; FAGNOU, K. ; ZUNIC, V.** *Org. Lett.,* 2002, vol. 4, 3465-3468
- **SNYDER, S. E. ; AVILES-GARAY, F. A. ; CHAKRABORTI, R. ; NICHOLS, D. E. ; WATTS, V. J. ; MAILMAN, R. B.** *J. Med. Chem.,* 1995, vol. 38, 2395-2409
- **KAMAL, A. ; GAYATRI, N. L.** *Tetrahedron Lett.,* 1996, vol. 37, 3359-3362
- **KIM, K. ; GUO, Y. ; SULIKOWSKI, G. A.** *J. Org. Chem.,* 1995, vol. 60, 6866
- **PERRONE, R. ; BERARDI, F. ; COLABUFO, N. A. ; LEOPOLDO, M. ; TORTORELLA, V. ; FIORENTINI, F. ; OLGIATI, V. ; GHIGLIERI, A. ; GOVONI, S.** *J. Med. Chem.,* 1995, vol. 3 (8), 942-949
- **CAPLE, R. ; CHEN, G. M.-S. ; NELSON, J. D.** *J. Org. Chem.,* 1971, vol. 36, 2874-2876
- **ARJONA, O. ; DE LA PRADILLA, R. F. ; GARCIA, E. ; MARTIN-DOMENECH, A. ; PLUMET, J.** *Tetrahedron Lett,* 1989, vol. 30, 6437-6440
- **LAUTENS, M. ; GAJDA, C. ; CHIU, P.** *J. Chem. Soc., Chem. Commun.,* 1993, 1193-1194
- **LAUTENS, M. ; SMITH, A. C. ; ABD-EL-AZIZ, A. S. ; HUBOUX, A. H.** *Tetrahedron Lett,* 1990, vol. 31, 3523
- **FUGAMI, K. ; HAGIWARA, S. ; ODA, H. ; KOSUGI, M.** *Synlett,* 1998, 477-478
- **MILLWARD, D. B. ; SAMMIS, G. ; WAYMOUTH, R. M.** *J. Org. Chem.,* 2000, vol. 65, 3902-3909
- **LAUTENS, M. ; HIEBERT, S. ; RENAUD, J.-L.** *Org. Lett.,* 2000, vol. 2, 1971-1973
- **LAUTENS, M. ; RENAUD, J.-L. ; HIEBERT, S.** *J. Am. Chem. Soc.,* 2000, vol. 122, 1804-1805
- **LAUTENS, M. ; HIEBERT, S. ; RENAUD, J.-L.** *J. Am. Chem. Soc.,* 2001, vol. 123, 6834-6839
- **RAYABARAPU, D. K. ; CHIOU, C.-F. ; CHENG, C.-H.** *Org. Lett.,* 2002, vol. 4, 1679-1682
- **MURAKAMI, M. ; IGAWA, H.** *Chem. Commun.,* 2002, 390-391
- **LAUTENS, M ; DOCKENDORFF, C. ; FAGNOU, K. ; MALICKI, A.** *Org. Lett.,* 2002, vol. 4, 1311-1314
- **LAUTENS, M. ; FAGNOU, K. ; ROVIS, T.** *J. Am. Chem. Soc.,* 2000, vol. 122, 5650
- **LAUTENS, M. ; FAGNOU, K. ; TAYLOR, M.** *Org. Lett.,* 2000, vol. 2, 1677
- **LAUTENS, M. ; FAGNOU, K. ; TAYLOR, M. ; ROVIS, T.** *J. Organomet. Chem.,* 2001, vol. 624, 259
- **LAUTENS, M. ; FAGNOU, K. ; HIEBERT, S.** *Acc. Chem. Res.,* 2003, vol. 36, 48
- **LAUTENS, M. ; FAGNOU, K. J.** *Am. Chem. Soc.,* 2001, vol. 123, 7170
- **LAUTENS, M. ; FAGNOU, K.** *Tetrahedron,* 2001, vol. 57, 5067
- **LAUTENS, M. ; FAGNOU, K. ; YANG, D.** *J. Am. Chem. Soc.,* 2003, vol. 125, 14884
- **LEONG, P. ; LAUTENS, M.** *J. Org. Chem.,* 2004, vol. 69, 2194
- **LAUTENS, M. ; HIEBERT, S. ; RENAUD, J.** *Org. Lett.,* 2000, vol. 2, 1971
- **CABRERA, S. ; ARRAYAS, R. G. ; CARRETERO, J. C.** *Angew. Chem., Int. Ed.,* 2004, vol. 43, 3944
- **LAUTENS, M. ; FAGNOU, K. ; ZUNIC, V.** *Org. Lett.,* 2002, vol. 4, 3465
- **CHO, Y-H. ; ZUNIC, V. ; SENBOKU, H. ; OLSEN, M. ; LAUTENS, M.** *J. Am. Chem. Soc.,* 2006, vol. 128, 6837
- *Tetrahedron Lett,* 2001, vol. 42, 223
- *J. Organomet. Chem.,* 2001, vol. 621, 89
- *Dalton Trans.,* 2003, 1606
- **GREENE ; WUTS.** Protective Groups in Organic Synthesis. John Wiley & Sons, Inc, 1999
- **ROBERT H. CRABTREE.** The Organometallic Chemistry of the Transition Metals. Wiley-Interscience, 2005
- Handbook of Reagents for Organic Synthesis. **LEO A. PAQUETTE.** Chiral Reagents for Asymmetric Synthesis. Wiley, 15 August 2003

- **PHAN T. B. ; BREUGST, M. ; MAYR, H.** *Angew. Chem. Int. Ed.,* 2006, vol. 45, 3869-3874
- **MAYR, H. ; PATZ, M.** *Angew. Chem. Int. Ed. Engl.,* 1994, vol. 33, 938-957